# EUROPEAN PATENT APPLICATION

(11) **EP 4 410 297 A1**
(43) Date of publication of application: **07.08.2024**
(21) Application number: 21958020.6
(22) Date of filing: 27.09.2021
(51) Int. Cl.: A61K 35/50, C12N 5/0775

(54) **DECIDUAL PLACENTAL MESENCHYMAL STEM CELL AND USE THEREOF IN PREPARATION OF PHARMACEUTICAL COMPOSITION FOR PROMOTING ANGIOGENESIS**

(71) Applicant: Vitaspring Biomedical Co., Ltd., New Taipei City, Taiwan 221416 (TW)
(72) Inventor: CHU, Li-Li, Taipei City, Taiwan 221416 (TW)
(74) Representative: Lang, Christian
(86) International application number: PCT/CN2021/120946
(87) International publication number: WO 2023/044902

(57) **Abstract**

Provided in the present invention are a decidual placental mesenchymal stem cell and a culture method therefor. The method comprises: in an existing culture method for a mesenchymal stem cell, an induction step is added, so that the decidual placental mesenchymal stem cell highly expresses Decoy receptor 3 (DcR3). Further provided in the present invention is the use of the decidual placental mesenchymal stem cell in the preparation of a drug for promoting angiogenesis.

## Description

### FIELD OF THE INVENTION

The present invention is related to a decidual placental mesenchymal stem cell and its culturing method, in particular, an induction step is added to currently existing mesenchymal stem cell culturing method, so that increasing the expression of Decoy receptor 3 (DcR3) in the decidual placental mesenchymal stem cell. The present invention is further related to a use of decidual placental mesenchymal stem cell for preparing pharmaceutical compositions for promoting angiogenesis.

### BACKGROUND OF THE INVENTION

In the present, the biomarkers used for identifying mesenchymal stem cells mostly refer to the standards published by International Society for Cell and Gene Therapy (ISCT): the cells must express CD73, CD90 and CD105 but do not express CD14, CD34, CD45 and HLA-DR. The combination of biomarkers is based on bone marrow mesenchymal stem cells, but the lack of tissue specificity makes the identification difficult to distinguish mesenchymal stem cells from different tissues. On the other hand, many reports pointed out that the behavior of mesenchymal stem cells from different sources is different. Therefore, it is necessary to develop specific biomolecular markers that distinguish mesenchymal stem cells from specific tissues.

Since a variety of angiogenesis factors expressed in mesenchymal stem cells, the cells are considered to be applied to promote angiogenesis to treat ischemic diseases. However, the mechanism of mesenchymal stem cells from different sources in promoting angiogenesis may be different due to the different performance of their key factors.

### DETAILED DESCRIPTION OF THE INVENTION

The present invention provides a method for culturing a decidual placental mesenchymal stem cells (pcMSCs) with highly expression of decoy receptor 3 (DcR3), which involves adding an induction step to a serum-free medium formulation (MCDB201 culture medium + EGF (epidermal growth factor) + ITS cell culture supplements (Insulin, Transferrin, Selenium)), wherein the said induction step is adding at least one cytokine to a cell culture medium for stimulation during the process of culturing, so that the pcMSCs highly express DcR3.

The term "highly express" or "high expression" of the invention means that the DcR3 expression level of the decidual placental mesenchymal stem cells cultured by the culture method of the present invention shows statistically significantly higher than that of pcMSCs cultured by the currently existing mesenchymal stem cell culture method.

In the method of the present invention, the at least one cytokine is preferably selected from Tumor Necrosis Factor-a (TNF-α), Interferon-γ (IFN-γ) or the combination thereof.

In the method of the present invention, the induction step is preferably performed after the pcMSCs adhere to the culture dish.

In the method of the present invention, most preferred, an effective amount of TNF-αis 15-20 ng/mL, and an effective amount of IFN-γ is 10-20 ng/mL.

In the method of the present invention, the stimulation of TNF-α and IFN-γ are most preferred performed for 48 hours.

In the method of the present invention, the TNF-α and the IFN-γ are most preferred added into the cell culture medium together.

A decidual placental mesenchymal stem cells (pcMSCs) cultured by the said culture method is also provided in the present invention, wherein the pcMSCs highly express Decoy receptor 3 (DcR3).

Preferably, the pcMSCs express estrogen receptor (ER), progesterone receptor (PR) and Decoy receptor 3 (DcR3).

To further investigate the applied potential of DcR3 in the pcMSCs, the present invention also comprising a use of pharmaceutical composition in the manufacturing of drug for promoting angiogenesis in a subject in need thereof, the said pharmaceutical composition comprises administering an effective amount of the pcMSCs.

Preferably, in the use of the present invention, wherein the subject is human or mammalian.

Preferably, in the use of the present invention, wherein the effective amount of pcMSCs is 1×10⁴ cells to 2×10⁵ cells.

### BRIEF DESCRIPTION OF THE DRAWINGS

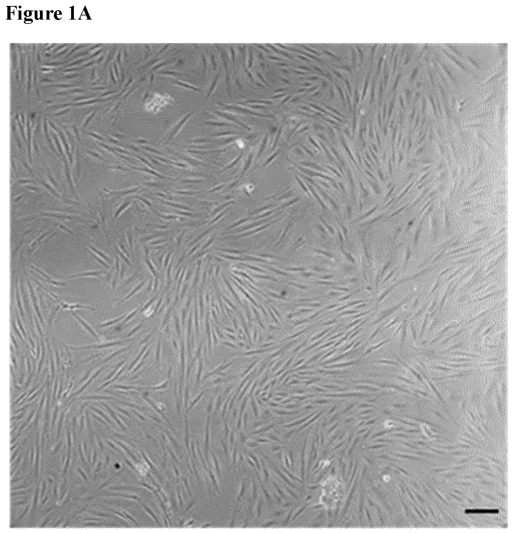

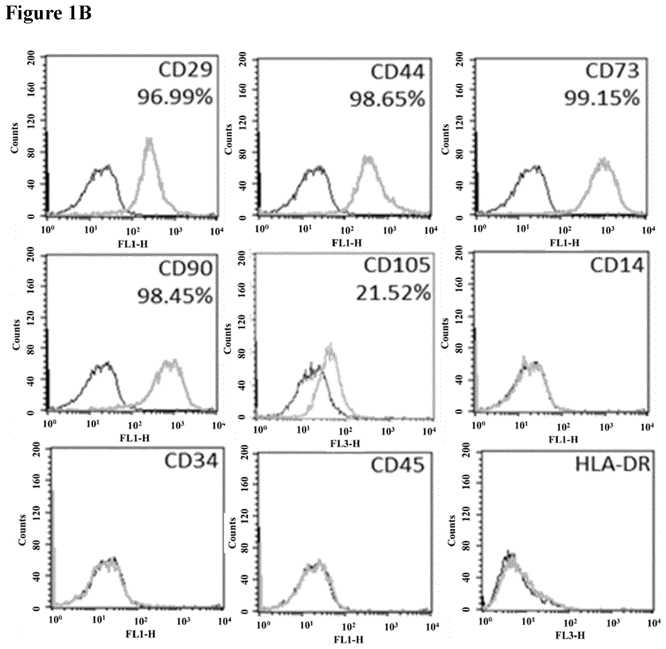

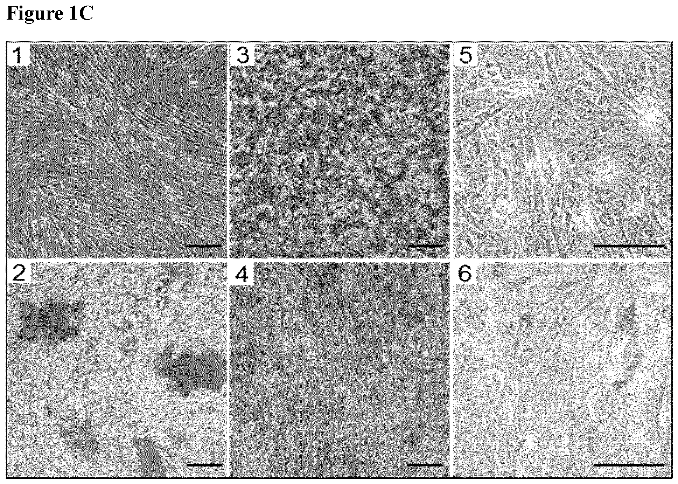

**Figure 1** depicts the characterization of pcMSCs. **(A)** Morphology of pcMSCs in serum free culture condition displayed spindle shape adherent cells. Scale bars: 100 µm. **(B)** Immunophenotype analysis of pcMSCs showed that biomarkers CD29, CD44, CD73, CD90, CD105 are exhibited positive, biomarkers CD14, CD34, CD45 and HLA-DR are exhibited negative. **(C)** In vitro differentiation abilities of pcMSCs in osteogenesis (C2), chondrogenesis (C4) and adipogenesis (C6) respectively. C1, C3 and C5 represent control group of each in vitro induction condition belonged to osteogenesis, chondrogenesis and adipogenesis respectively. Scale bars: 200 µm.

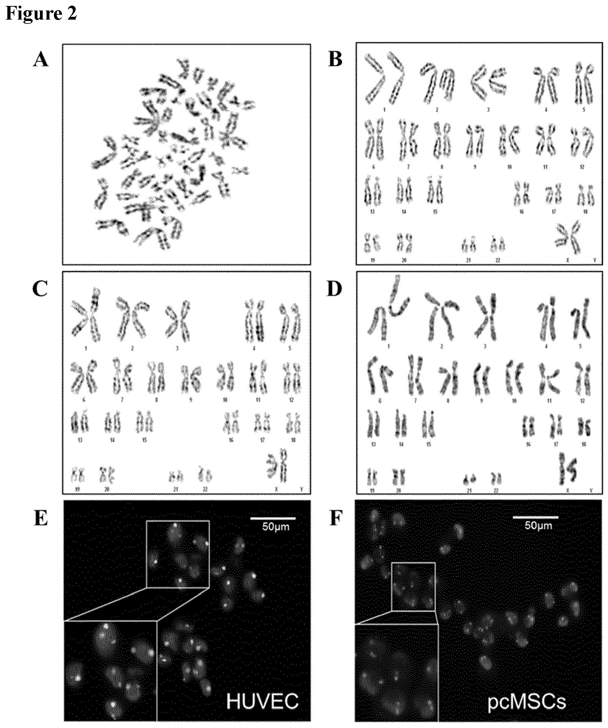

**Figure 2** demonstrates the karyotypically characteristics of pcMSCs isolated from male newborn's placenta. **(A)** Representative image of chromosome analysis of 20^{th} passage pcMSCs (N100). The analysis presented as 46 independent chromosomes of three individuals including N98 **(B),** N99 **(C),** and N100 **(D).** Fluorescence in situ hybridization of pcMSCs isolated from male newborn's placenta showed X chromosome (represented as red fluorescence) positive but Y chromosome (represented as green fluorescence) negative **(F),** in contrast to that in Human umbilical vein endothelial cell (HUVEC) from male newborn presenting double positive result of X and Y chromosome **(E).** Given at the left-bottom corners are the enlarged views of the images in white boxes of the individual panels. Scale bars: 50 µm.
**Figure 3** demonstrates the gene expression analysis of pcMSCs comparing to bone marrow mesenchymal stem cells (BMMSC). Abbreviation: pcMSCs: decidual placental mesenchymal stem cell; BMMSC: bone marrow mesenchymal stem cells; Adsv: Adipose-derived mesenchymal stem cells; hES: human embryonic stem cells; UniRef: UniProt Reference Clusters.
**Figure 4** shows hormone receptors of pcMSCs comparing to those of BMMSCs. Abbreviation: PRA: progesterone receptor A; PRB: progesterone receptor B; ER: estrogen receptor α subunit.
**Figure 5** indicates decidualization of pcMSCs can be induced by in vitro induction of estradiol and progesterone. **(A)** The result of PCR quantitative analysis. **(B)** The result of Western Blot quantitative analysis. **(C)** The result of ELISA quantitative analysis. * means 0.01 *< P* ≤0.05, ** means 0.005 < *P* ≤ 0.01, *** means *P* ≤ 0.005; and N.D. means non-detected.
**Figure 6** indicates the comparison of TNF receptor superfamily associated genes expression level of pcMSCs and BMMSCs.
**Figure 7** demonstrates the result of protein expression level of DcR3 in pcMSCs and BMMSCs.
**Figure 8** demonstrates the expression level of DcR3 is enhanced by the induction of TNF-α and IFN-γ.
**Figure 9** illustrates the experiment processes of *in vitro* tube formation assay of pcMSCs by DcR3.
**Figure 10** illustrates the results of *in vitro* tube formation assay demonstrated pcMSCs promote angiogenesis by DcR3. **(A)** The result of ELISA anakysis. **(B)** The images taken by fluorescent microscope. **(C)** The statistical results of images taken by fluorescent microscope. The result is presented as mean ± SD (n=3), * means 0.01 < *P* ≤ 0.05; ** means 0.005 < *P* ≤ 0.01; *** means *P <* 0.005.

### EMBODIMENTS

It should be understood that the detailed description of the embodiments is to illustrate the preferred embodiments of the present invention, and is not intended to limit the present invention to certain embodiments. It should be noted that the present invention is intended to cover all alternative embodiments within the same spirit and scope of the present invention. Some nonessential modifications and adjustments made by others based on the concept of the present invention still belong to the protection scope of the present invention.

### Example 1. Identification of decidual placental mesenchymal stem cells (pcMSCs)

In the research of the present invention, a kind of mesenchymal stem cells was successfully isolated from human placental decidua, named placenta choriodecidual-derived mesenchymal stromal cells (pcMSCs).

A kind of mesenchymal stem cells was isolated from the choriodecidual part of the placenta, which meet the basic international definition of mesenchymal stem cells (Figure 1), and then the cells were confirmed to originate from the maternal site (which is the decidua site) by chromosome identification and fluorescence in situ heterozygous staining analysis (Figure 2).

Since the basic definition of mesenchymal stem cells could not distinguish cells separated from different tissue sources, in order to find a biomarker sufficient to identify decidual mesenchymal stem cells, the gene expression of bone marrow mesenchymal stem cells and decidual mesenchymal stem cells were analyzed in the present invention.

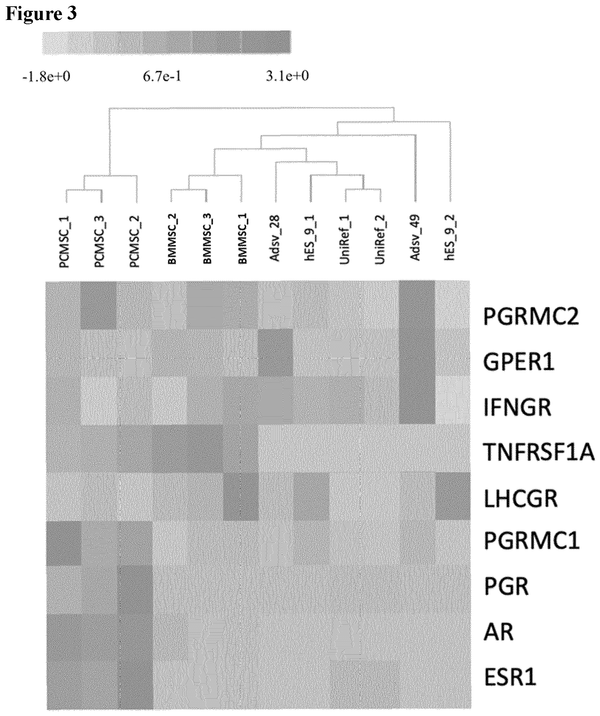

Several receptor genes were collected in the present invention to prove the difference between bone marrow mesenchymal stem cells (BMMSCs) and pcMSCs. As shown in Figure 3, the pcMSCs expressed estrogen receptor (ESR) and progesterone receptor (PGR) which were not expressed in BMMSCs, and both BMMSCs and pcMSCs expressed IFN-γ receptor (IFNGR) and TNF-α receptor (TNFRSF1A). The result confirmed that ESR and PGR were unique to decidual mesenchymal stem cells.

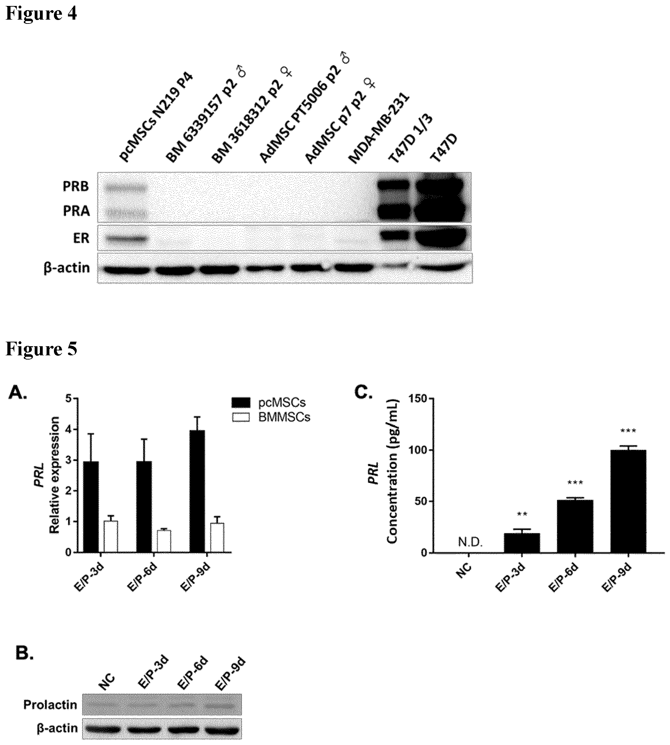

Further compared the hormone receptors of pcMSCs with those of BMMSC, the protein expression was confirmed by the Western Blot with different receptor specific antibodies, and the breast cancer cell lines MDA-MB-231 and T47D were used as negative and positive controls of progesterone receptor (PR) and estrogen receptor (ER), respectively. The result demonstrated that, after compared to BMMSCs (BM) and Adipose-derived mesenchymal stem cells (AdMSCs), only pcMSCs expressed progesterone receptor (PR) and estrogen receptor (ER) (see Figure 4).

In order to identify that estrogen receptor and progesterone receptor were unique to decidual mesenchymal stem cells, the decidual mesenchymal stem cells (pcMSCs) and bone marrow mesenchymal stem cells (BMMSCs) were further stimulated with 10 nM estrogen and 1 µM progesterone (E/P), and the cultured medium and the cells were harvested on day 3, 6 and 9 for subsequently quantitative PCR (qPCR), Western Blot and Enzyme-linked immunosorbent assay (ELISA) quantitative analysis to evaluate the expression of prolactin (PRL). β-actin was used as quantitative standards in Western Blot, all experiments were repeated in triplicate.

The results were shown as Figure 5. Regardless of the results of qPCR **(A),** Western Blot **(B)** or ELISA **(C)** quantitative analysis demonstrated that only the pcMSCs could differentiate into decidualization , the index gene PRL rose significantly. The BMMSCs were not regulated by hormones. The results confirmed once again that decidual mesenchymal stem cells exhibited their unique behaviors derived from the decidual site.

Moreover, the expression levels of Tumor Necrosis Factor (TNF) receptor superfamily related genes of pcMSCs and BMMSCs were also compared. The Tumor Necrosis Factor (TNF) receptor superfamily related genes were collected and analyzed to evaluate the specific gene expression patterns between pcMSCs and BMMSCs.

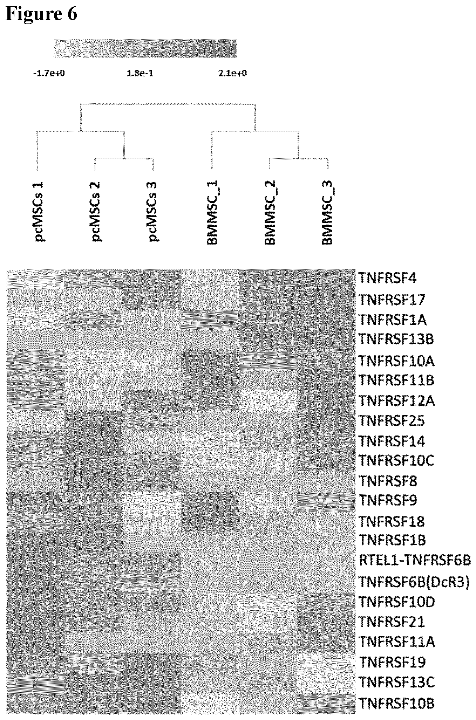

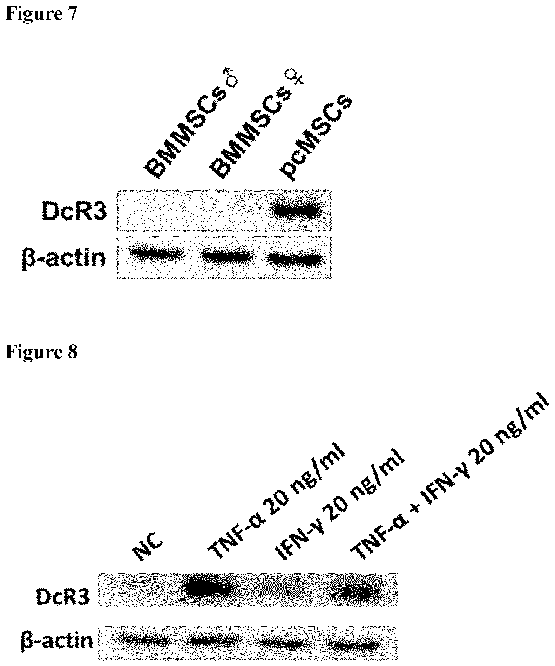

Although several genes showed distinguishable patterns in pcMSCs (see Figure 6), but the decoy receptor 3 (DcR3; or TNFRSF6B) was more focused and further analyzed to confirm that DcR3 was expressed only in pcMSCs but not in BMMSCs. As shown in Figure 7, the DcR3 was expressed in pcMSCs but was not expressed in BMMSCs isolated from different genders. Since that, estrogen receptor (ER), progesterone receptor (PR), and decoy receptor type 3 (DcR3) were considered to be able to become physiologically significant biomarkers of pcMSCs to be used to distinguish the mesenchymal stem cell derived from different tissues.

### Example 2. Method for culturing a decidual placental mesenchymal stem cells (pcMSCs) with highly expression of DcR3

The present invention provided a new method for culturing a decidual placental mesenchymal stem cells (pcMSCs) which was to add inflammation-related cytokines TNF-α and IFN-γ into the conventional mesenchymal stem cell culture method.

In detail, the pcMSCs were cultured by a conventional sterilized cell culture method with using serum-free stem cell culture medium for cell subculture, the fresh stem cell culture medium was renewed every 3 days during the culturing, wherein the medium including MCDB201 formulation culture medium, 1% Insulin-Transferrin-Selenium (ITS) and 10 ng/mL epidermal growth factor (EGF).

Differing from the conventional culturing method, an induction step was added in the present invention after the pcMSCs adhered to the culture dish.

In the present invention, the cells were separated to 4 groups, that were (1) negative control; (2) induction with 20ng/mL TNF-α; (3) induction with 20ng/mL IFN-γ; and (4) induction with 20ng/mL TNF-α and 20ng/mL IFN-γ simultaneously.

At first, 1×10⁵ pcMSCs were cultured in 6-well culture plate, after overnight culturing in incubator, the culture medium of each well was removed, and each well was respectively supplied (1) complete medium without stimulated factor, (2) complete medium with 20ng/mL TNF-α, (3) complete medium with 20ng/mL IFN-γ, and (4) complete medium having 20ng/mL TNF-α and 20ng/mL IFN-γ according to the condition of each group. The pcMSCs were cultured for 48 hours and then were identified by Western Blot after induction. The results indicated that the expression level of DcR3 was increased in pcMSCs of all the cytokine induced groups (see Figure 8).

The present invention further tested the applicable dosage of cytokines for induction of pcMSCs to increase the expression level of DcR3. The result showed that the DcR3 expression level of pcMSCs could also be increased by using 15ng/mL TNF-α, and the DcR3 expression level of pcMSCs could be increased by using 10ng/mL IFN-γ. Similarly, when those two cytokines were added together for the induction, the result of increasing the expression level of DcR3 in pcMSCs was also observed.

### Example 3. pcMSCs had the potential to promote angiogenesis

To further explore the application potential of DcR3 in decidual mesenchymal stem cells (pcMSCs), the pcMSCs were used in the present invention for the research.

The present invention further tested the difference of angiogenesis between pcMSCs with or without induction of TNF-α and IFN-γ.

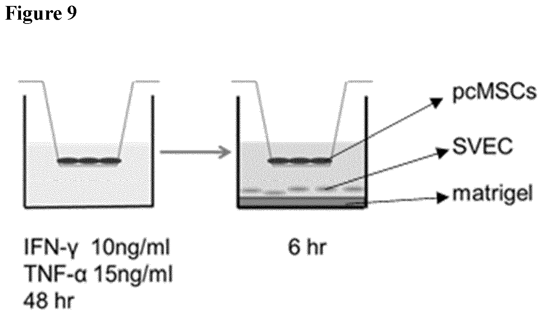

According to the culturing method described previously, the pcMSCs were cultured in Transwell inserts, the procedures of the experiment were shown in Figure 9. The used cell number was adjusted according to the diameters of the Transwell inserts.

First of all, 1×10⁴ pcMSCs were cultured in Transwell inserts specific to 24-well culture dish. After induction with 15ng/mL TNF-α and 10ng/mL IFN-γ for 48 hours, the Transwell inserts were moved into the culture dishes with endothelial cell line (SVEC), the induced pcMSCs of the present invention were co-cultured with SVEC for *in vitro* tube formation assay. The SVECs were cultured on the Matrigel cell culture matrix. The amount of total branch point was calculated with images captured by fluorescence microscopy after 6 hours of co-culturing.

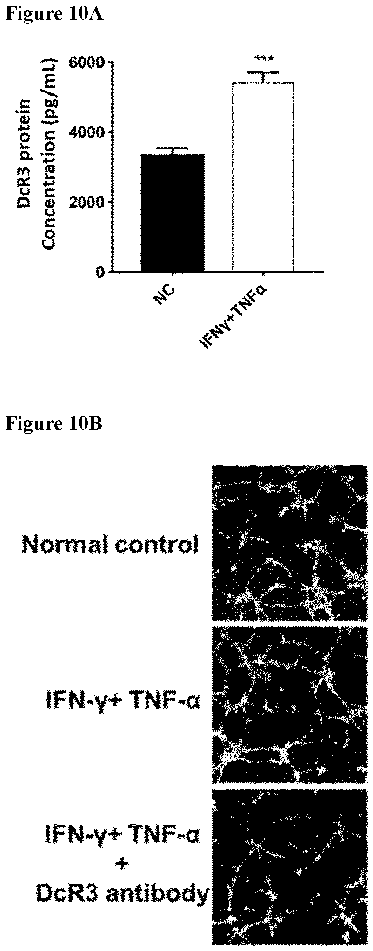

The DcR3 concentration of the cultured medium were evaluated by ELISA analysis after 48 hours induction of pcMSCs of the present invention with cytokines. The DcR3 expression level of induced group was significantly higher than the expression level of non-induced group (as shown in Figure 10A).

The pcMSCs of the present invention and the SVEC were co-cultured for 6 hours, the number of total branch points were calculated through the images taken by the fluorescence microscope. The represnetive image of each group was shown in Figure 10B.

In the present invention, 2×10⁵ pcMSCs were also cultured in Transwell inserts specific to 6-well culture dish to the same experiments, and similar results were also obtained (data not shown).

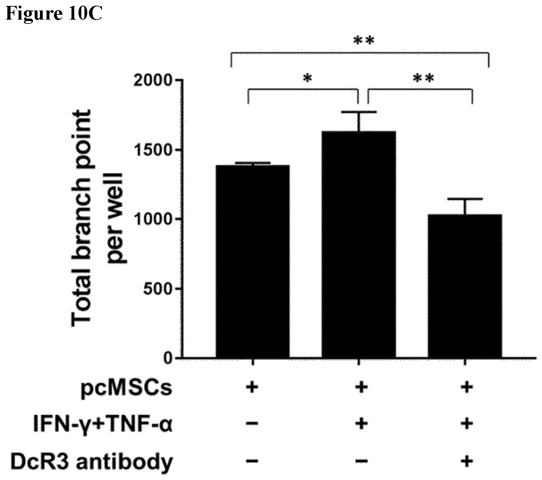

The statistical results showed that, compared with the normal control group, pcMSCs induced with TNF-α and IFN-γ significantly induced more branch points, and this phenomenon was reversed by using anti-DcR3 antibody (0.5 µg/mL) (see Figure 10C). The number of branch points in the neutralization group of anti-DcR3 antibody showed the lowest, even lower than that of the normal control group. The results reflected that the constant expression of DcR3 had an impact on angiogenesis. Therefore, pcMSCs could promote angiogenesis by expressing DcR3.

## Claims

1. A method for culturing decidual placental mesenchymal stem cells (pcMSCs), **characterized in that**, the said method comprises adding an induction step into a serum-free medium formulation, wherein the said induction step is to add at least one cytokine into the cell medium for stimulation during the culturing process, so that the pcMSCs highly express Decoy receptor 3 (DcR3); the said serum-free medium formulation comprising MCDB201 medium, estrogen receptor and insulin-transferrin-selenium (ITS) cell culture supplements.

2. The method of claim 1, **characterized in that**, the at least one cytokine is selected from Tumor Necrosis Factor-α (TNF-α), Interferon-γ (IFN-γ), or the combination thereof.

3. The method of claim 1, **characterized in that**, the induction step is performed by adding at least one cytokine after the pcMSCs adhere to the culture dish.

4. The method of claim 2, **characterized in that**, the effective amount of TNF-α is 15-20 ng/mL, and the effective amount of IFN-γ is 10-20 ng/mL.

5. The method of claim 2, **characterized in that**, the stimulation of TNF-α and IFN-γ are performed for 48 hours.

6. The method of claim 2, **characterized in that**, the TNF-α and the IFN-γ are added into the cell culture medium together.

7. A decidual placental mesenchymal stem cells (pcMSCs) cultured by the method of claim 1, **characterized in that**, the said pcMSCs highly express Decoy receptor 3 (DcR3).

8. The pcMSCs of claim 7, **characterized in that**, the said pcMSCs express estrogen receptor (ER), progesterone receptor (PR) and DcR3.

9. A use of pharmaceutical composition in the manufacturing of drug for promoting angiogenesis, **characterized in that**, the pharmaceutical composition comprises an effective amount of the decidual placental mesenchymal stem cells (pcMSCs) of claim 7.

10. The use of claim 9, **characterized in that**, the said pcMSCs express estrogen receptor (ER), progesterone receptor (PR) and Decoy receptor 3 (DcR3).

11. The use of claim 9, **characterized in that**, the effective amount of pcMSCs is 1×10⁴ cells ~ 2×10⁵ cells.
